# EUROPÄISCHE PATENTANMELDUNG

(11) **EP 1 489 142 A1**
(43) Veröffentlichungstag der Anmeldung: **22.12.2004**
(21) Anmeldenummer: 04022499.0
(22) Anmeldetag: 07.02.2002
(51) Int. Cl.: C09B 57/00, A61K 7/13

(54) **Kationische Farbstoffe, Verfahren zu deren Herstellung und diese Verbindungen enthaltende Färbemittel**

(30) Priorität: 22.06.2001 DE 10130145
(62) Teilanmeldung aus: 02712898.2
(71) Anmelder: Wella Aktiengesellschaft, 64274 Darmstadt (DE)
(72) Erfinder: Sauter, Guido, Dr., 3174 Thörishaus (CH); Braun, Hans-Jürgen, Dr., 3182 Ueberstorf (CH); Brouillard, Raymond, Prof., 67200 Strasbourg (FR); Fougerousse, André, 67440 Birkenwald (FR); Roehri-Stoeckel, Christine, Dr., 67000 Strasbourg (FR); Gonzalez, Emmanuel, Dr., 21000 Dijon (FR)

(57) **Zusammenfassung**

Die vorliegende Erfindung betrifft Verbindungen der Formel (V), ein Verfahren zu deren Herstellung sowie die Verbindungen der Formel (V) enthaltende Färbemittel.

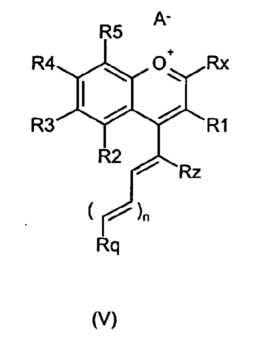

## Beschreibung

Gegenstand der vorliegenden Erfindung sind neue kationische Farbstoffe, ein Verfahren zu deren Herstellung und diese kationischen Farbstoffe enthaltende Färbemittel.

Aus der Literatur, beispielsweise J.G. Sweeny und G.A. lacobucci in Tetrahedron, 37, 1481-1483 (1981), G. A. lacobucci und J. G. Sweeny in Tetrahedron, 39, 3005-3038 (1983) und von M. Elhabiri, P. Figuereido, A. Fougerousse und R. Brouillard in Tetrahedron Lett. 36, 4611-4614 (1995), sind bereits verschiedenene Syntheseverfahren für bestimmte 4-Alkylen-2-aryl-4H-1-benzopyran-Derivate und deren Salzen bekannt. Ein oft angewendetes Verfarhren ist zum Beispiel eine Kondensationsreaktion zwischen einem 2-Hydroxybenzaldehyd-Derivat mit einem Acetophenon-Derivat welche zu den 4-Alkylen-2-aryl-4H-1-benzopyran-Derivaten führt. In einem anderen Verfahren werden reduzierende oder oxidative Reagentien mit diversen Flavonoidverbindungen zur Reaktion gebracht. Diese Verfahren sind jedoch nicht in jeder Hinsicht befriedigend.

Überraschenderweise wurde nunmehr gefunden, dass 4-Alkylen-2-aryl-4H-1-benzopyran-Derivate der Formel (la) oder deren Salze der Formel (I), welche auf einfache Weise durch Umsetzung der Flavon-verbindungen der Formel (II) mit einer Grignard-Verbindung der Formel (III) hergestellt werden können, als Ausgangsstoffe für die Herstellung von neuen kationischen Farbstoffen der Formel (V) hervorragend geeignet sind.

Die Herstellung der als Ausgangsverbindungen dienenden 4-Alkylen-2-aryl-4H-1-benzopyran-Derivate der Formel (la) beziehungsweise deren Salzen der Formel (I), 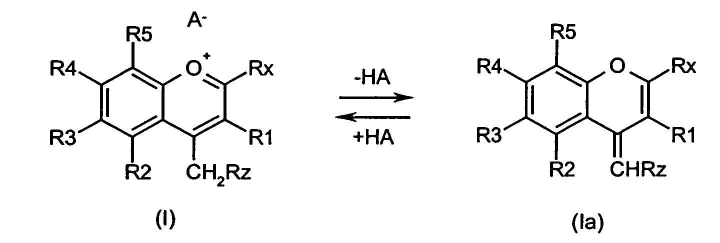 erfolgt durch Umsetzung eines Mols einer Flavonverbindung der Formel (II) in einem apolar aprotischen oder polar aprotischen Lösungsmittel mit 1 bis 50 Mol einer Verbindung der Formel (III) bei einer Temperatur von -80 bis 180 °C und anschließende Behandlung des Reaktionsproduktes mit einer sauren, wässrigen oder wässrig-alkoholischen Lösung; 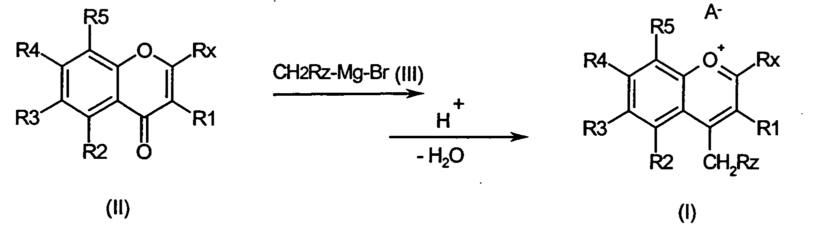 wobei gilt:
**R1, R2, R3, R4,** und **R5** sind unabhängig voneinander gleich einem Wasserstoffatom, einer geradkettigen oder verzweigten C1- bis C4-Alkylgruppe, einer C1- bis C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethylgruppe, einer -CHO -Gruppe, einer -COR^{b}-Gruppe, einer -COOH -Gruppe, einer -CO₂R^{b}-Gruppe, einer -OCOR^{b}-Gruppe, einer -OCH₂Aryl-Gruppe, einer -SO₂NH₂-Gruppe, einer -SO₂CHF₂-Gruppe, einer -SO₂CF₃-Gruppe, einer -SO₂NH₂-Gruppe, einer -SO₂NHR^{b}-Gruppe, einer -SO₂N(R^{b})₂-Gruppe, einer -SO₂R⁴-Gruppe, einer -NH₂-Gruppe, einer -(NH₃)⁺-Gruppe, einer -NHR^{b}-Gruppe, einer -(NH₂R^{b})⁺-Gruppe, einer -N(R^{b})₂-Gruppe, einer -(N(R^{b})₃)⁺-Gruppe, einer -NHCOR^{b}-Gruppe, einer -NHCOOR^{b}-Gruppe, einer -CH₂NH₂-Gruppe, einer -CH₂NHR^{b}-Gruppe, einer -CH₂N(R^{b})₂-Gruppe, einer -CO₂CF₃-Gruppe oder einer -PO(OR^{b})₂-Gruppe, wobei **R**^{**b**} gleich einem Wasserstoffatom, einem gegebenenfalls substituierten aromatischen Carbozyklus oder Heterozyklus oder einer C1- bis C6-Alkylgruppe ist;
Rx ist gleich einem Wasserstoffatom, einer gegebenfalls substituierten Phenylgruppe, einer gegebenfalls substituierten Naphtylgruppe, einer Benzylgruppe, einem gegebenenfalls substituierten aromatischen Carbozyklus oder Heterozyklus, einer C1- bis C8-Polyhydroxyalkylgruppe, einer C1- bis C8-Alkoxy-(C1 bis C8)alkylgruppe, einer geradkettigen oder verzweigten C1-C6-Alkylgruppe, einer OR^{a}-Gruppe oder einer SR^{a}-Gruppe;
**Rz** ist gleich einem Wasserstoffatom, einer gegebenfalls substituierten Phenylgruppe, einer gegebenfalls substituierten Naphtylgruppe, einem gegebenenfalls substituierten aromatischen Carbozyklus oder Heterozyklus, einer C1- bis C8-Alkylgruppe, einer C1- bis C8-Monohydroxyalkylgruppe, einer C1- bis C8-Polyhydroxyalkylgruppe, einer C1- bis C8-Alkoxy-(C1 bis C8)alkylgruppe, einem Halogenatom, einer -OCOR^{a}-Gruppe, einer Nitrogruppe, einer Cyanogruppe, einer -CO-R^{a}-Gruppe, einer -CO-OR^{a}-Gruppe, einer -CO-OCF₃-Gruppe, einer -CO-NHR^{a}-Gruppe, einer -CO-N(R^{a})₂-Gruppe, einer -SO₂-NH₂-Gruppe, einer -SO₂-NHR^{a}-Gruppe, einer -SO₂-N(R^{a})₂, einer -SO₂-OR^{a}-Gruppe oder einer -SO₂-R^{a}-Gruppe, wobei **R**^{**a**} gleich einem Wasserstoffatom, einem gegebenenfalls substituierten aromatischen Carbozyklus oder Heterozyklus oder einer C1-C6-Alkylgruppe ist;
**A**^{**-**} ist gleich dem Anion einer organischen oder anorganischen Säure, vorzugsweise einem Chlorid, Bromid, Jodid, Hydrogensulfat, Sulfat, Toluolsulfonat, Benzolsulfonat, Monomethylsulfat, Hexafluorophosphat, Hexafluorantimonat, Tetrafluoroborat, Tetraphenylborat, Formiat, Acetat oder Propionat, wobei das Chloridion, dasTetrafluoroboration, das Acetation und das Hydrogensulfation besonders bevorzugt sind.

Die Grignardreaktion zwischen der Verbindungen der Formel (II) und der Formel (III) erfolgt bei -80 bis 180 °C , vorzugsweise 0 bis 140 °C und insbesondere 20 bis 100 °C, unter Verwendung eines apolar aprotischen oder polar aprotischen Lösungsmittels, beispielsweise einer Etherverbindung, vorzugsweise Diethylether, Dioxan, 1,2-Dimethoxyethan, 1,2-Diethoxyethan, Bis-(2-ethoxyethyl)ether und insbesondere Tetrahydrofuran. Das Reaktionsmedium ist wasserfrei.

Die Reaktionsdauer beträgt etwa 1 bis 48 Stunden, wobei eine Reaktionszeit von 1 bis 8 Stunden und insbesondere 2 bis 6 Stunden bevorzugt ist.

Die Verbindung der Formel (II) und die Verbindung der Formel (III) werden vorzugsweise in einem molaren Verhältnis von 1:1 bis 1:50 und insbesondere in einem molaren Verhältnis von 1:1 bis 1:15 eingesetzt. Die Aufarbeitung der Grignardreaktion erfolgt durch Zugabe der Reaktionslösung in eine durch eine Säure (HA) sauer eingestellte wässrige oder wässrig-alkoholische Lösung, vozugsweise in eine mit NH₄Cl Salz gesättigte und zusätzlich mit 37%iger HCl-Lösung sauer eingestellten (pH = 1-5; vorzugsweise pH = 2-4)) Wasserphase. Je nach Löslichkeit kann die Verbindung der Formel (I) oder (la) nach der Wasserabspaltung bereits ausfallen. Andernfalls wird die Wasserphase mit für diesen Zweck üblichen organischen Lösungsmitteln, beispielsweise halogenierten Kohlenwasserstoffen, Etherverbindungen, Esterverbindungen oder einer überkritischen Flüssigkeit aus CO₂, vorzugsweise Etherverbindungen oder Esterverbindungen und insbesondere Essigsäureethylester, extrahiert. Der so erhaltene Extrakt wird sodann in eine mit einer Säure sauer eingestellten wässrig-alkoholischen Lösung aufgenommen und bei einer Temperatur von 0° bis 100 °C, wobei eine Temperatur von 20° bis 50° besonders bevorzugt ist, gerührt. Das Rühren erfolgt vorzugsweise für 1 bis 48 Stunden und insbesondere 1 bis 8 Stunden, wobei die Verbindung der Formel (I) oder (la) erhalten wird.

Als Verbindungen der Formeln (I) und (la) können insbesondere das 7-Hydroxy-4-methylen-2-phenyl-4H-1-benzopyran, 7-Methoxy-4-methylen-2-(3,4,5-trimethoxyphenyl)-4H-1-benzopyran, 5,7-Dimethoxy-4-methylen-2-phenyl-4H-1-benzopyran, 4-Methylen-4H-1-benzopyran, 4-Methylen-2-phenyl-4H-1-benzopyran, 7-Amino-4-methylen-2-phenyl-4H-1-benzopyran, 7-Dimethylamino-4-methylen-2-phenyl-4H-1-benzopyran und 4-Methylen-7-nitro-2-phenyl-4H-1-benzopyran sowie deren Salze mit anorganischen oder organischen Säuren genannt werden; wobei das 7-Hydroxy-4-methyl-2-phenyl-1-benzopyrylium-chlorid, 7-Methoxy-4-methyl-2-(3,4,5-trimethoxyphenyl)-1-benzopyrylium-chlorid, 5,7-Dimethoxy-4-methyl-2-phenyl-1-benzopyrylium-chlorid, 4-Methyl-1-benzopyryliumchlorid, 4-Methyl-2-phenyl-1-benzopyrylium-chlorid, 7-Amino-4-methyl-2-phenyl-1-benzopyrylium-chlorid, 7-Dimethylamino-4-methyl-2-phenyl-1-benzopyrylium-chlorid und 4-Methyl-7-nitro-2-phenyl-1-benzopyryliumchlorid besonders bevorzugt sind.

Die vorliegende Erfindung betrifft ein Verfahren zur Herstellung von kationischen Farbstoffen der Formel (V), bei dem das vorstehend beschriebene 4-Alkylen-2-aryl-4H-1-benzopyran-Derivat der Formel (la) oder dessen Salz der Formel (I) mit einem Aldehyd-Derivat der Formel (IV), vorzugsweise 4-N,N-Dimethylamino-benzaldehyd oder 4-N,N-Dimethylaminocinnamaldehyd, bei 0 bis 180 °C in einem polar aprotischen, apolar aprotischen, polar protischen oder apolar protischen Lösungsmittel, umgesetzt wird; 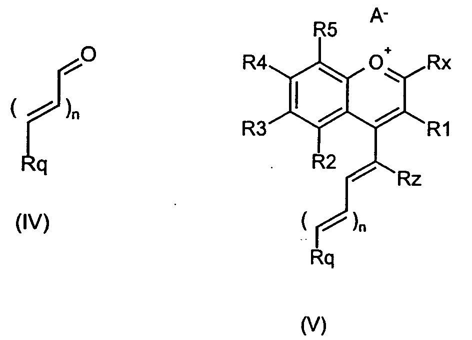 wobei gilt:
**R1, R2, R3, R4,** und **R5** sind unabhängig voneinander gleich einem Wasserstoffatom, einer geradkettigen oder verzweigten C1- bis C4-Alkylgruppe, einer C1- bis C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethylgruppe, einer -CHO -Gruppe, einer -COR^{b}-Gruppe, einer -COOH-Gruppe, einer -CO₂R^{b}-Gruppe, einer -OCOR^{b}-Gruppe, einer -OCH₂Aryl-Gruppe, einer -SO₂NH₂ -Gruppe, einer -SO₂CHF₂-Gruppe, einer -SO₂CF₃-Gruppe, einer -SO₂NH₂-Gruppe, einer -SO₂NHR^{b}-Gruppe, einer -SO₂N(R^{b})₂-Gruppe, einer -SO₂R^{b}-Gruppe, einer -NH₂ -Gruppe, einer -(NH₃)⁺ -Gruppe, einer -NHR^{b} -Gruppe, einer -(NH₂R^{b})⁺ -Gruppe, einer -N(R^{b})₂-Gruppe, einer -(N(R^{b})₃)⁺-Gruppe, einer -NHCOR^{b}-Gruppe, einer -NHCOOR^{b}-Gruppe, einer -CH₂NH₂-Gruppe, einer -CH₂NHR^{b}-Gruppe, einer -CH₂N(R^{b})₂-Gruppe, einer -CO₂CF₃-Gruppe oder einer -PO(OR^{b})₂-Gruppe, wobei **R**^{**b**} gleich einem Wasserstoffatom, einem gegebenenfalls substituierten aromatischen Carbozyklus oder Heterozyklus oder einer C1- bis C6-Alkylgruppe ist;
Rq ist gleich einer substituierten Pyridylgruppe, einer substituierten Pyrimidylgruppe, einer Phenylgruppe der Formel (VI) oder einem heterozyklischen Rest der Formel (VII) 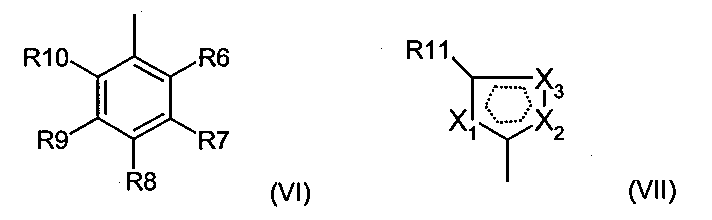 mit
R6, R7, R8, R9 und R10 sind unabhängig voneinander gleich einem Wasserstoffatom, einer geradkettigen oder verzweigten C1-C4-Alkylgruppe, einer C1-C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethyl-gruppe, einer -CHO -Gruppe, einer -COR^{c}-Gruppe, einer -COOH -Gruppe, einer -CO₂R^{c}-Gruppe, einer -OCOR^{c}-Gruppe, einer -OCH₂Aryl-Gruppe, einer -SO₂NH₂-Gruppe, einer -NH₂-Gruppe, einer -(NH₃)⁺-Gruppe, einer -NHR^{c}-Gruppe, einer -(NH₂R^{c})⁺ -Gruppe, einer -N(R^{c})₂-Gruppe, einer -(N(R^{c})₃)⁺-Gruppe, einer -NHCOR^{c}-Gruppe, einer -NHCOOR^{c}-Gruppe, einer -CH₂NH₂-Gruppe, einer -CH₂NHR^{c}-Gruppe, einer -CH₂N(R^{c})₂-Gruppe, einer -CO₂CF₃-Gruppe, einer -PO(OR^{c})₂ -Gruppe, einer -SO₂CHF₂ -Gruppe, einer -SO₂CF₃-Gruppe, einer -SO₂R^{c}- Gruppe oder einer -SR^{c}-Gruppe,
wobei **R**^{**c**} gleich einem Wasserstoffatom, einem gegebenenfalls substituierten aromatischen Carbozyklus oder Heterozyklus oder einer C1- C6-Alkylgruppe ist, mit der Massgabe, das zwei nebeneinanderliegende Reste R6 bis R10 auch gemeinsam mit den Kohlenstoffatomen des aromatischen Kerns einen 5- oder 6gliedrigen alizyklischen oder aromatischen Ring bilden können, der gegebenenfalls ein oder mehrere Schwefelatome, Stickstoffatome und/oder Sauerstoffatome enthalten kann; und
**X1** ist gleich Schwefel, Stickstoff, Sauerstoff, einer C-R13-Gruppe oder einer N-R12-Gruppe;
**X2** ist gleich Schwefel, Stickstoff, Sauerstoff, einer C-R14-Gruppe oder einer N-R12-Gruppe;
**X3** ist gleich Schwefel, Stickstoff, Sauerstoff, einer C-R15-Gruppe oder einer N-R12-Gruppe,
unter der Bedingung, dass mindestens einer und maximal zwei der Reste **X1** bis **X3** gleich Schwefel, Sauerstoff oder einer N-R12-Gruppe ist.
**R11, R13, R14** und **R15** sind unabhängig voneinander gleich Wasserstoff, einem Halogenatom (F, Cl, Br, J) einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄-Hydroxyalkylaminogruppe, einer Trifluormethangruppe, einer -C(O)CH₃-Gruppe, einer -C(O)CF₃-Gruppe, einer -Si(CH₃)₃-Gruppe, einer C₁-C₄-Hydroxyalkylgruppe oder einer C₃-C₄-Dihydroxyalkylgruppe; und
**R12** ist gleich Wasserstoff, einer C₁-C₆-Alkylgruppe, einer C₂-C₄-Hydroxyalkylgruppe, einer Phenylgruppe oder einer Acetylgruppe; n ist gleich 0, 1 oder 2;
**Rx** ist gleich einem Wasserstoffatom, einer gegebenfalls substituierten Phenylgruppe, einer gegebenfalls substituierten Naphtylgruppe, einer Benzylgruppe, einem gegebenenfalls substituierten aromatischen Carbozyklus oder Heterozyklus, einer C1- bis C8-Polyhydroxyalkylgruppe, einer C1- bis C8-Alkoxy-(C1 bis C8)alkylgruppe, einer geradkettigen oder verzweigten C1-C6-Alkylgruppe, einer OR^{a}-Gruppe oder einer SR^{a}-Gruppe,
wobei **R**^{**a**} gleich einem Wasserstoffatom, einem gegebenenfalls substituierten aromatischen Carbozyklus oder Heterozyklus oder einer C1-C6-Alkylgruppe ist;
**Rz** ist gleich einem Wasserstoffatom, einer gegebenfalls substituierten Phenylgruppe, einer gegebenfalls substituierten Naphtylgruppe, einem gegebenenfalls substituierten aromatischen Carbozyklus oder Heterozyklus, einer C1- bis C8-Alkylgruppe, einer C1- bis C8-Monohydroxyalkylgruppe, einer C1- bis C8-Polyhydroxyalkylgruppe, einer C1- bis C8-Alkoxy-(C1 bis C8)alkylgruppe, einem Halogenatom, einer -OCOR^{a}-Gruppe, einer Nitrogruppe, einer Cyanogruppe, einer -CO-R^{a}-Gruppe, einer -CO-OR^{a}-Gruppe, einer -CO-OCF₃-Gruppe, einer -CO-NHR^{a}-Gruppe, einer -CO-N(R^{a})₂-Gruppe, einer -SO₂-NH₂-Gruppe, einer -SO₂-NHR^{a}-Gruppe, einer -SO₂-N(R^{a})₂, einer -SO₂-OR^{a}-Gruppe oder einer -SO₂-R^{a}-Gruppe, wobei **R**^{**a**} gleich einem Wasserstoffatom, einem gegebenenfalls substituierten aromatischen Carbozyklus oder Heterozyklus oder einer C1-C6-Alkylgruppe ist; und
**A**^{**-**} ist gleich dem Anion einer organischen oder anorganischen Säure, vorzugsweise einem Chlorid, Bromid, Jodid, Hydrogensulfat, Sulfat, Toluolsulfonat, Benzolsulfonat, Monomethylsulfat, Hexafluorophosphat, Hexafluorantimonat, Tetrafluoroborat, Tetraphenylborat, Formiat, Acetat oder Propionat, wobei das Chloridion, dasTetrafluoroboration, das Acetation und das Hydrogensulfation besonders bevorzugt sind;
unter der Bedingung, dass Rq nicht gleich einer unsubstituierten Phenylgruppe oder unsubstituierten Thienylgruppe ist, wenn gilt n=0, **R1=R2=R3=R4=R5=Rz=H** und **Rx** gleich einer unsubstituierten Phenylgruppe.

Die Kondensationsreaktion erfolgt vorzugsweise bei 20 bis 140 °C und insbesondere bei 50 bis 100 °C, wobei die Verwendung eines polar protischen oder apolar protischen Lösungsmittel, wie zum Beispiel Wasser, Alkoholen wie Ethanol und Methanol, oder Carbonsäuren wie Essigsäure und Ameisensäure, bevorzugt ist. Besonders bevorzugt ist hierbei die Verwendung von Ethanol und/oder Methanol.
Die Reaktionsdauer beträgt vorzugsweise etwa 1 bis 48 Stunden und insbesondere 1 bis 8 Stunden.

Das Syntheseverfahren für die Farbstoffe der Formel (V) wird in Gegenwart von Luftsauerstoff durchgeführt. Die Reaktion ist in dem nachfolgenden Schema 1 zusammengefasst.

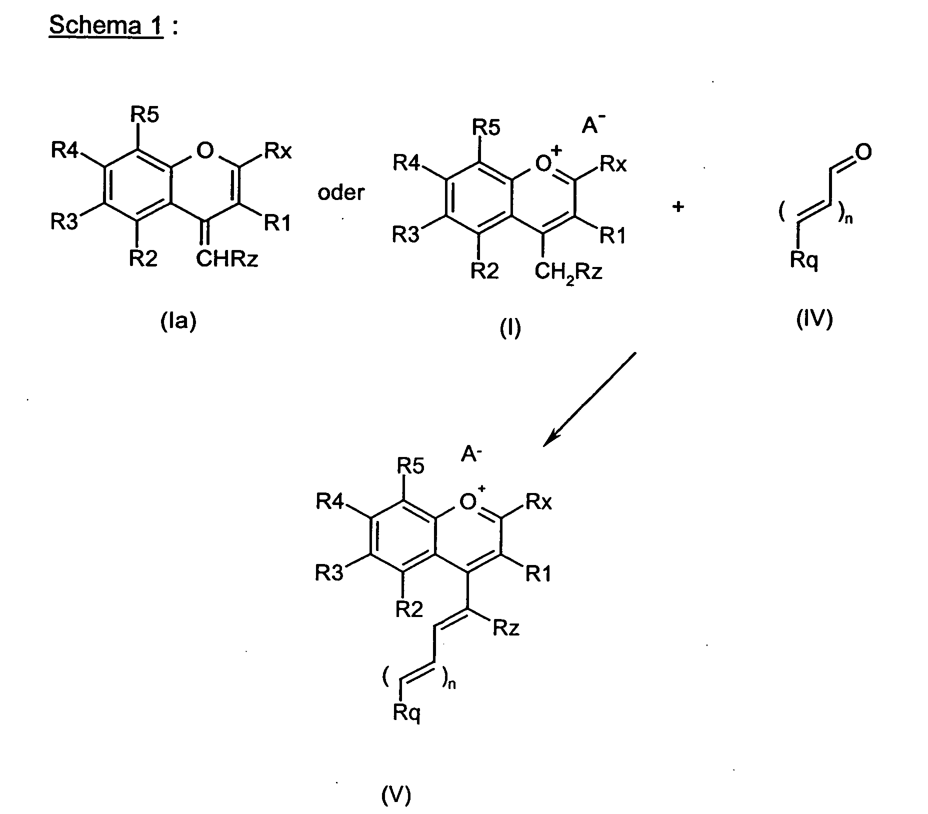

Ein weiterer Gegenstand sind die neuen kationischen Farbstoffe der Formel (V).

Als geeignete Verbindungen der Formel (V) können hierbei insbesondere genannt werden: 4-[4-(4-N,N-dimethylamino)phenyl)-ethenyl]-7-hydroxy-2-phenyl-1-benzopyrylium-chlorid, 4-[4-(4-(N,N-dimethylamino)phenyl)ethenyl]-7-methoxy-2-(3,4,5-trimethoxyphenyl)-1-benzopyryliumchorid und 4-[4-(4-(N,N-dimethylamino)phenyl)-butadienyl]-7-methoxy-2-(3,4,5-trimethoxy phenyl)-1-benzopyrylium-chlorid.

Die Farbstoffe der Formel (V) eignen sich hervorragend als direktziehende Farbstoffe zum Färben von Keratinfasern.

Ein weiterer Gegenstand der vorliegenden Erfindung ist daher ein Mittel zur Färbung von Keratinfasern, beispiesweise Wolle, Baumwolle, Seide oder Haaren, insbesondere menschlicher Haare, welches dadurch gekennzeichnet ist, dass es mindestens eine Verbindung der Formel (V) enthält.

Der Gesamtgehalt an den Farbstoffen der Formel (V) beträgt in dem erfindungsmässen Mittel zur Färbung von Keratinfasern etwa 0,01 bis 5 Gewichtsprozent, vorzugsweise 0,1 bis 3 Gewichstprozent.

Weiterhin kann das erfindungsgemäße Färbemittel gegebenenfalls zusätzlich übliche, physiologisch unbedenkliche, direktziehende Farbstoffe aus der Gruppe der sauren oder basischen Farbstoffe, Nitrofarbstoffe, Azofarbstoffe, Chinonfarbstoffe und Triphenylmethan-farbstoffe enthalten.

Das erfindungsgemäße Mittel zur Färbung von Keratinfasern kann beispielsweise eine Lösung, insbesondere eine wässrige oder wässrig-alkoholische Lösung sein. Weitere geeignete Zubereitungsformen sind eine Creme, ein Gel, ein Schaum oder eine Emulsion. Ihre Zusammensetzung stellt eine Mischung der Verbindungen der Formel (V) mit den für solche Zubereitungen üblichen Zusätzen dar.

Übliche in Färbemitteln verwendete Zusätze in Lösungen, Cremes, Emulsionen, Gelen oder Schäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 30 Gewichtsprozent (bezogen auf die Farbträgermasse) und die Pflegestoffe in einer Konzentration von etwa 0,1 bis 5 Gewichtsprozent (bezogen auf die Farbträgermasse).

Ebenfalls ist es möglich, dieses Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder als Aerosolschaum aus einem Druckbehälter zu entnehmen.

Der pH-Wert des erfindungsgemäßen Färbemittels beträgt etwa 2 bis 11, wobei ein pH-Wert von 2,5 bis 8 besonders bevorzugt ist. Die Einstellung eines alkalischen pH-Wertes erfolgt vorzugsweise mit Ammoniak, es ist jedoch auch möglich, anstelle von Ammoniak organische Amine, wie zum Beispiel Monoethanolamin oder Triethanolamin zu verwenden. Für die Einstellung eines sauren pH-Wertes kann hingegen eine organische oder anorganische Säure, wie zum Beispiel Salzsäure, Schwefelsäure, Phosphorsäure, Ascorbinsäure, Glycolsäure oder Milchsäure, verwendet werden.

Selbstverständlich kann das vorstehend beschriebene Färbemittel gegebenenfalls weitere für Färbemittel für Keratinfasern übliche Zusätze, wie zum Beispiel Pflegestoffe, Netzmittel, Verdicker, Weichmacher, Konservierungsstoffe und Parfümöle sowie auch weitere, nachstehend aufgeführte Zusätze enthalten.

Weiterhin können in dem erfindungsgemäßen Färbemittel Netzmittel oder Emulgatoren aus den Klassen der anionischen, amphoteren, nichtionogenen oder zwitterionischen oberflächenaktiven Substanzen wie Fettalkoholsulfate, Alkansulfonate, Alkylbenzolsulfonate, Alkylbetaine, α-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkernmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein.

Das vorstehend beschriebene Färbemittel kann weiterhin natürliche oder synthetische Polymere beziehungsweise modifizierte Polymere natürlichen Ursprungs enthalten sein, wodurch gleichzeitig mit der Färbung eine Festigung der Keratinfaser erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent. Die vorgenannten Polymere können in dem erfindungsgemäßen Mittel in der für solche Mittel üblichen Menge, insbesondere in einer Menge von etwa 1 bis 5 Gewichts-prozent, verwendet werden.

Das erfindungsgemäße Mittel zur Färbung von Keratinfasern eignet sich insbesondere zu Färbung von Haaren. Das erfindungsgemäße Färbemittel wird hierzu in üblicher Weise in einer für die Färbung der Haare ausreichende Menge, im allgemeinen etwa 50 bis 150 Gramm, auf das Haar aufgetragen. Nach einer für die Haarfärbung ausreichenden Einwirkungszeit, die üblicherweise etwa 10 bis 45 Minuten bei 20 bis 50 °C, vorzugsweise 15 bis 30 Minuten bei etwa 40°C, beträgt, wird das Haar mit Wasser gespült, gegebenenfalls mit einem Shampoo gewaschen und/oder mit der wäßrigen Lösung einer schwachen organischen Säure, wie zum Beispiel Zitronensäure oder Weinsäure, nachgespült und getrocknet.

Die Anwendung des Färbemittels mit zusätzlicher Festigung erfolgt in bekannter und üblicher Weise durch Befeuchten des Haares mit dem Festiger, Einlegen des Haares zur Frisur und anschließende Trocknung.

Das erfindungsgemäße Färbemittel ergibt auch ohne den Zusatz von Oxidationsmitteln Ausfärbungen mit hervorragender Brillianz und Farbtiefe. Obwohl eine Verwendung des vorgenannten Mittels ohne den Zusatz von Oxidationsmitteln wegen der besseren Schonung der Faser bevorzugt ist, ist eine Verwendung des vorgenannten Färbemittels in Verbindung mit Oxidationsmitteln ohne weiteres möglich, beispielsweise wenn eine gleichzeitige Bleichung der Faser gewünscht wird oder wenn dem Färbemittel auch übliche Oxidationsfarbstoffvorstufen zugesetzt werden sollen.

Hinsichtlich der färberischen Möglichkeiten bietet das erfindungsgemäße Färbemittel je nach Art und Zusammensetzung der verwendeten Farbstoffe eine breite Palette verschiedener Farbnuancen, die sich von natürlichen Farbtönen bis hin zu hochmodischen, leuchtenden Nuancen erstreckt. Die vorzüglichen Eigenschaften des neuen Färbemittels zeigen sich insbesondere auf durch Licht und Wetter geschädigtem oder auf dauergewellten Haaren. Hierbei zeichnen sich die erhaltenen Färbungen insbesondere auch durch ihre sehr gute Haltbarkeit und Auswaschbeständigkeit aus.

Die nachstehenden Beispiele sollen den Erfindungsgegenstand näher erläutern, ohne ihn auf die angeführten Beispiele zu beschränken.

### Beispiele

### Beispiel 1: Synthese der 4-Alkyl-2-aryl-1-benzopyrylium-Salze der Formel (la) = 7-Methoxy-4-methyl-2-(3,4,5-trimethoxyphenyl)-1-benzopyrylium-chlorid

Zu 1,6 ml einer 3M Methylmagnesium Bromid-THF-Lösung wird langsam eine Lösung von 0,4 g 7,3',4',5'-Tetramethoxyflavon in 15 ml trockenem THF getropft. Anschließend wird die Reaktionslösung 2 Stunden lang unter Rückfluss erhitzt und sodann zu 50 ml einer gesättigten NH₄Cl-Lösung gegeben. Die Wasserphase wird mit Essigsäureethylester extrahiert. Die vereinigten organischen Phasen werden über Na₂SO₄ getrocknet, filtritriert und vollständig eingeengt. Der so erhaltene Rückstand wird in saurem Methanol (Methanol mit 2-3 Tropfen 37%iger HCl) aufgenommen und 1 Stunde lang bei Raumtemperatur gerührt. Das Lösungsmittel wird unter vermindertem Druck vollständig eingeengt, wobei ein roter Feststoff erhalten wird.
Ausbeute: 0,42 g (95 % der Theorie) 7-Methoxy-4-methyl-2-(3,4,5-trimethoxyphenyl)-1-benzopyrylium-chlorid
¹H-NMR (CD₃OD): δ = 8,68 ppm (s, 1 H); 8,43 ppm (d; J = 9 Hz, 1H); 8,03 ppm (d, J = 2 Hz, 1 H); 7,84 ppm (s, 2H); 7,59 ppm (dd, J = 9 Hz; J = 2Hz, 1H); 4,22 ppm (s, 3H); 4,08 (s, 6H); 4,00 ppm (s, 3H);
Hochauflösendes Massenspektrum:
M⁺ berechnet für C₂₀H₂₁O₅: 341,1389; gefunden: 341,1417
UV-VIS (HCI 0,1 M mit 1% MeOH): 444 nm (ε = 28200 ± 500);

### Beispiel 2.1 bis 2.3: Synthese der kationischen Farbstoffe der Formel (V)

### Beispiel 2.1: 4-[4-(4-(N,N-dimethylamino)phenyl)ethenyl]-7-hydroxy-2-phenyl-1-benzopyrylium-chlorid

0,40 g 7-Hydroxy-4-methyl-2-phenyl-1-benzopyrylium-chlorid und 0,24 g 4-N,N-Dimethylaminobenzaldehyd werden in Methanol gelöst und für drei Stunden unter Rückfluss erhitzt. Anschließend wird die Reaktionsmischung vollständig eingeengt. Der erhaltene grüne Rückstand wird mit Diethylether/Essigsäureethylester (1/1) gewaschen und in Ethanol umkristallisiert.
Ausbeute: 0,50 g (84 % der Theorie) 4-[4-(4-(N,N-dimethylamino)phenyl)ethenyl]-7-hydroxy-2-phenyl-1-benzopyrylium-chlorid
¹H-NMR (CD₃OD): δ = 8,42 ppm (d, J = 15 Hz, 1 H); 8,39 ppm (d, J = 9 Hz, 1 H); 8,25 ppm (d, J = 7,5 Hz, 2H); 8,14 ppm (s, 1 H); 7,87 ppm (d, J = 9 Hz, 2H); 7,76 ppm (d, J = 15 Hz, 1 H); 7,72-7,61 ppm (m, 3H); 7,07 ppm (d, J = 9 Hz, 1 H); 6,97 ppm (s, 1 H); 6,86 ppm (d, J = 9 Hz, 2H); 3,17 ppm (s, 6H).
Hochauflösendes Massenspektrum:
M⁺ berechnet für C₂₅H₂₂NO₂: 368,1650; gefunden: 368,1654
UV-VIS (MeOH mit 5% HCl 0.1 M): 640 nm (ε = 80000 ± 1000);

### Beispiel 2.2: 4-[4-(4-(N,N-dimethylamino)phenyl)ethenyl]-7-methoxy-2-(3,4,5-trimethoxyphenyl)-1-benzopyrylium-chorid

0,18 g 7-Methoxy-4-methyl-2-(3,4,5-trimethoxyphenyl)-1-benzopyryliumchlorid und 0,078 g 4-N,N-Dimethylaminobenzaldehyd werden in Methanol gelöst und drei Stunden lang unter Rückfluss erhitzt. Anschließend wird die Reaktionsmischung vollständig eingeengt. Der erhaltene grüne Rückstand wird mit Diethylether/Essigsäureethylester (1/1) gewaschen und in Ethanol umkristallisiert.
Ausbeute: 0,20 g (80 % der Theorie) 4-[4-(4-(N,N-dimethylamino)phenyl)ethenyl]-7-methoxy-2-(3,4,5-trimethoxyphenyl)-1-benzopyrylium-chorid
¹H-NMR (CD₃OD): δ = 8,69 ppm (d, J = 15 Hz, 1 H); 8,47 ppm (d, J = 9 Hz, 1 H); 8,25 (s, 1 H); 7,95 ppm (d, J = 9 Hz, 2H); 7,72 ppm (d, J = 15 Hz, 1 H); 7,58 ppm (s, 2H); 7,51 ppm (d, J = 2 Hz, 1 H); 7,28 ppm (dd, J = 9 Hz; J = 2 Hz, 1 H); 6,92 ppm (d, J = 9 Hz, 2H); 4,08 ppm (s, 3H); 4,06 ppm (s, 6H); 3,95 ppm (s, 3H); 3,29 ppm (s, 6H)
Hochauflösendes Massenspektrum:
M⁺ berechnet für C₂₉H₃₀NO₅: 472,2124; gefunden: 472,2125
UV-VIS (MeOH mit 5% HCl 0,1 M): 652 nm (ε = 78600 ± 800);

### Beispiel 2.3: 4-[4-(4-(N,N-dimethylamino)phenyl)-butadienyl]-7-methoxy-2-(3,4,5-trimethoxy phenyl)-1-benzopyrylium-chlorid

0,15 g 7-Methoxy-4-methyl-2-(3,4,5-trimethoxyphenyl)-1-benzopyryliumchlorid und 0,077 g 4-N,N-Dimethylaminöcinnamaldehyd werden in Methanol 3 Stunden lang unter Rückfluss erhitzt. Anschließend wird die Reaktionsmischung vollständig eingeengt. Der erhaltene grüne Rückstand wird chromatografisch an Silikagel mit MeOH/CH₂Cl₂ (5/95) als Laufmittel gereinigt.
Ausbeute: 0,16 g (75 % der Theorie) 4-[4-(4-(N,N-dimethylamino)phenyl)butadienyl]-7-methoxy-2-(3,4,5-trimethoxy phenyl)-1-benzopyryliumchlorid
¹H-NMR (CD₃OD mit CF₃COOD): δ = 8,62 ppm (dd, J = 15 Hz, J = 12 Hz, 1 H); 8,39 ppm (d, J = 9 Hz, 1 H); 8,38 ppm (s, 1 H); 7,72 ppm (d, J = 9 Hz, 2H); 7,65 ppm (s, 2H); 7,61 ppm (d, J = 15 Hz, 1 H); 7,59 ppm (d, J = 2 Hz, 1 H); 7,53 ppm (d, J = 15 Hz, 1 H); 7,45 ppm (dd, J = 15 Hz; J = 12 Hz, 1 H); 7,34 ppm (dd, J = 9 Hz, J = 2 Hz, 1 H); 7,10 ppm (d, J = 9 Hz, 2H); 4,07 ppm (s, 3H); 4,03 ppm (s, 6H); 3,92 ppm (s, 3H); 3,21 ppm (s, 6H).
Hochauflösendes Massenspektrum:
M⁺ berechnet für C₃₁H₃₂NO₅: 498,2280; gefunden: 498,2291
UV-VIS (MeOH mit 5% HCl 0,1 M): 730 nm (ε = 66600 ± 900).

### Beispiel 3.1 bis 3.9: Färbemittel für Keratinfasern

### Beispiel 3.1: Haarfärbemittel

| | |
|---|---|
| 0,05 g | 4-[4-(4-N,N-dimethylamino)phenyl)ethenyl]-7-hydroxy-2-phenyl-1-benzopyrylium-chlorid |
| 50,00 g | Ethanol |
| ad 100,00 g | Wasser, demineralisiert |

Die obige Färbelösung wird mit 10%iger Zitronensäurelösung oder mit 0,1 M NaOH-Lösung auf den in den Beispielen 3.1. a-d angegebenen pHₘ-Wert eingestellt und anschließend auf gebleichtes Haar aufgetragen.

Nach einer Einwirkungszeit von 30 Minuten bei 40°C wird mit Wasser ausgewaschen und getrocknet.

| | | | | **Farbmeßwerte** | | |
|---|---|---|---|---|---|---|
| **Beispiel Nr.** | | **Farbton nach der Färbung** | | **L** | **a** | **b** |
| | | | unbehandelte Haare : | 63,30 | -0,48 | +10,40 |
| **3.1.a** | pHₘ= 4,9 | violett | Nach dem Färben : | 31,10 | +7,61 | -8,41 |
| **3.1.b** | pHₘ= 3,2 | violett | Nach dem Färben : | 34,89 | +7,08 | -10,89 |
| **3.1.c** | pHₘ= 6,4 | violett | Nach dem Färben : | 32,51 | +9,00 | -11,06 |
| **3.1.d** | pHₘ= 9,1 | violett | Nach dem Färben : | 33,61 | +8,60 | -10,75 |

### Beispiel 3.2: Haarfärbemittel

| | |
|---|---|
| 0,20 g | 4-[4-(4-(N,N-dimethylamino)phenyl)ethenyl]-7-hydroxy-2-phenyl-1-benzopyrylium-chlorid |
| 1,60 g | Decylglucosid (Plantacare 2000 UPder Firma Fluka) |
| 0,08 g | Ethylendiaminoteraacetat-Dinatriumsalz |
| 62,00 g | Ethanol |
| ad 100,00 g | Wasser, demineralisiert |

Die obige Färbelösung wurde auf gebleichtes Haar (L= 63,3; a = -0,48; b= 10,4) aufgetragen. Nach einer Einwirkungszeit von 30 Minuten bei 40°C wird mit Wasser ausgewaschen und getrocknet. Es wir eine violette Färbung erhalten (L= 33,97; a= 7,35; b= -10,88).

### Beispiel 3.3: Haarfärbemittel

| | |
|---|---|
| 0,05 g | 4-[4-(4-(N,N-dimethylamino)phenyl)ethenyl]-7-methoxy-2-(3,4,5-trimethoxyphenyl)-1-benzopyrylium-chorid |
| 50,00 g | Ethanol |
| ad 100,00 g | Wasser, demineralisiert |

Die obige Färbelösung wird auf gebleichtes Haar (L= 63,3; a = -0,48; b= 10,4) aufgetragen. Nach einer Einwirkungszeit von 30 Minuten bei 40°C wird mit Wasser ausgewaschen und getrocknet. Es wird eine blaugrüne Färbung erhalten (L= 39,22; a= -10,15; b= -8,19).

### Beispiel 3.4: Haarfärbemittel

| | |
|---|---|
| 0,320 g | 4-[4-(4-(N,N-dimethylamino)phenyl)ethenyl]-7-methoxy-2-(3,4,5-trimethoxyphenyl)-1-benzopyrylium-chlorid |
| 1,410 g | Cocamidopropylbetain |
| 0,014 g | Ameisensäure |
| 40,600 g | Ethanol |
| ad 100,000 g | Wasser, demineralisiert |

Die obige Färbelösung wird auf gebleichtes Haar (L= 63,3; a = -0,48; b= 10,4) aufgetragen. Nach einer Einwirkungszeit von 30 Minuten bei 40°C wird mit Wasser ausgewaschen und getrocknet. Es wird eine violette Färbung erhalten (L= 39,50; a= 2,19; b= -8,31).

### Beispiel 3.5: Haarfärbemittel

| | |
|---|---|
| 0,05 g | 4-[4-(4-(N,N-dimethylamino)phenyl)-butadienyl]-7-methoxy-2-(3,4,5-trimethoxy phenyl)-1-benzopyrylium-chlorid |
| 50,00 g | Ethanol |
| ad 100,00 g | Wasser, demineralisiert |

Die obige Färbelösung wird auf gebleichtes Haar (L= 63,3; a = -0,48; b= 10,4) aufgetragen. Nach einer Einwirkungszeit von 30 Minuten bei 40°C wird mit Wasser ausgewaschen und getrocknet. Es wird eine grüne Färbung erhalten (L= 42,26; a= -20,94; b= 0,59).

### Beispiel 3.6: Haarfärbemittel

| | |
|---|---|
| 0,27 g | 4-[4-(4-(N,N-dimethylamino)phenyl)-butadienyl]-7-methoxy-2-(3,4,5-trimethoxy phenyl)-1-benzopyrylium-chlorid |
| 2,00 g | Decylglucosid (Plantacare 2000 UP der Firma Fluka) |
| 0,10 g | Ethylendiaminoteraacetat-Dinatriumsalz |
| 52,50 g | Ethanol |
| ad 100,00 g | Wasser, demineralisiert |

Die obige Färbelösung wird auf gebleichtes Haar (L= 63,3; a = -0,48; b= 10,4) aufgetragen. Nach einer Einwirkungszeit von 30 Minuten bei 40°C wird mit Wasser ausgewaschen und getrocknet. Es wird eine blaugrüne Färbung erhalten (L= 35,40; a= -18,42; b= 2,12).

### Beispiel 3.7: Haarfärbemittel

| | |
|---|---|
| 0,01 g | 7-Hydroxy-2-phenyl-4-[(E)-2-(2-thienyl)ethenyl]-1-benzopyrilium-chlorid |
| 50,00 g | Ethanol |
| ad 100,00 g | Wasser, demineralisiert |

Die obige Färbelösung wird mit 10%iger Zitronensäurelösung oder mit 0,1 M NaOH-Lösung auf den in den Beispielen 3.7. a-d angegebenen pHₘ-Wert eingestellt und anschließend auf gebleichtes Haar aufgetragen. Nach einer Einwirkungszeit von 30 Minuten bei 40°C wird mit Wasser ausgewaschen und getrocknet.

| | | | | **Farbmeßwerte** | | |
|---|---|---|---|---|---|---|
| **Beispiel Nr.** | | **Farbton nach der Färbung** | | **L** | **a** | **b** |
| | | | unbehandelte Haare : | 63,30 | -0,48 | +10,40 |
| **3.7.a** | pHₘ= 3,8 | orange | Nach dem Färben : | 66,64 | +21,24 | +19,15 |
| **3.7.b** | pHₘ= 2,9 | orange | Nach dem Färben : | 63,55 | +25,43 | +23,55 |
| **3.7.c** | pHₘ= 6,0 | orange | Nach dem Färben: | 68,75 | +19,44 | +18,93 |
| **3.7.d** | pHₘ= 9,6 | orange | Nach dem Färben: | 67,97 | +21,08 | +19,15 |

### Beispiel 3.8: Haarfärbemittel

| | |
|---|---|
| 0,01 g | 4-[(E)-2-(2-furyl)ethenyl]-7-hydroxy-2-phenyl-1-benzopyrilium chlorid |
| 50,00 g | Ethanol |
| ad 100,00 g | Wasser, demineralisiert |

Die obige Färbelösung wird mit 10%iger Zitronensäurelösung oder mit 0,1 M NaOH-Lösung auf den in den Beispielen 3.8. a-d angegebenen pHₘ-Wert eingestellt und anschließend auf gebleichtes Haar aufgetragen. Nach einer Einwirkungszeit von 30 Minuten bei 40°C wird mit Wasser ausgewaschen und getrocknet.

| | | | | **Farbmeßwerte** | | |
|---|---|---|---|---|---|---|
| **Beispiel Nr.** | | **Farbton nach der Färbung** | | **L** | **a** | **b** |
| | | | unbehandelte Haare : | 63,30 | -0,48 | +10,40 |
| **3.8.a** | pHₘ= 3,6 | orange | Nach dem Färben : | 60,08 | +19,60 | +19,55 |
| **3.8.b** | pHₘ= 2,8 | orange | Nach dem Färben : | 51.41 | +24,76 | +24,30 |
| **3.8.c** | pHₘ= 6,0 | orange | Nach dem Färben : | 66,81 | +19,33 | +19,52 |
| **3.8.d** | pHₘ= 9,5 | orange | Nach dem Färben: | 64,94 | +19,40 | +18,62 |

### Beispiel 3.9: Haarfärbemittel

| | |
|---|---|
| 0,01 g | 4-[(E)-2-(2,4-dihydroxyphenyl)ethenyl]-7-hydroxy-2-phenyl-1-benzopyrilium-chlorid |
| 50,00 g | Ethanol |
| ad 100,00 g | Wasser, demineralisiert |

Die obige Färbelösung wird mit 10%iger Zitronensäurelösung oder mit 0,1 M NaOH-Lösung auf den in den Beispielen 3.9. a-d angegebenen pHₘ-Wert eingestellt und anschließend auf gebleichtes Haar aufgetragen. Nach einer Einwirkungszeit von 30 Minuten bei 40°C wird mit Wasser ausgewaschen und getrocknet.

| | | | | **Farbmeßwerte** | | |
|---|---|---|---|---|---|---|
| **Beispiel Nr.** | | **Farbton nach der Färbung** | | **L** | **a** | **b** |
| | | | unbehandelte Haare : | 63,30 | -0,48 | +10,40 |
| **3.9.a** | pHₘ= 4,0 | rot | Nach dem Färben : | 58.34 | +14,71 | +6,44 |
| **3.9.b** | pHₘ= 3,0 | rot | Nach dem Färben : | 36,63 | +15,13 | +2,70 |
| 3.9.c | pHₘ= 6,1 | rot | Nach dem Färben : | 65,38 | +12,78 | +6,52 |
| 3.9.d | pHₘ= 9,1 | rot | Nach dem Färben : | 54,45 | +8,24 | +2,77 |

Alle Prozentangaben stellen, soweit nicht anders angegeben, Gewichtsprozente dar.

## Patentansprüche

1. Kationischer Farbstoff der Formel (V), 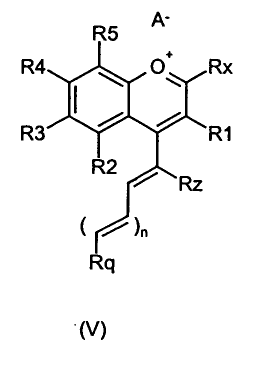 wobei gilt:
**R1, R2, R3, R4,** und **R5** sind unabhängig voneinander gleich einem Wasserstoffatom, einer geradkettigen oder verzweigten C1- bis C4-Alkylgruppe, einer C1- bis C4-Hydroxyalkylgruppe, einer Hydroxygruppe, einer Methoxygruppe, einer Benzylgruppe, einem Halogenatom, einer Nitrogruppe, einer Nitrosogruppe, einer Cyanogruppe, einer Trifluormethylgruppe, einer -CHO -Gruppe, einer -COR^{b}-Gruppe, einer -COOH-Gruppe, einer -CO₂R^{b}-Gruppe, einer -OCOR^{b}-Gruppe, einer -OCH₂Aryl-Gruppe, einer -SO₂NH₂-Gruppe, einer -SO₂CHF₂-Gruppe, einer -SO₂CF₃ -Gruppe, einer -SO₂NH₂-Gruppe, einer -SO₂NHR^{b}-Gruppe, einer -SO₂N(R^{b})₂-Gruppe, einer -SO₂R^{b}-Gruppe, einer -NH₂-Gruppe, einer -(NH₃)⁺-Gruppe, einer -NHR^{b}-Gruppe, einer -(NH₂R^{b})⁺-Gruppe, einer -N(R^{b})₂-Gruppe, einer -(N(R^{b})₃)⁺-Gruppe, einer -NHCOR^{b}-Gruppe, einer -NHCOOR^{b}-Gruppe, einer -CH₂NH₂-Gruppe, einer -CH₂NHR^{b}-Gruppe, einer -CH₂N(R^{b})₂-Gruppe, einer -CO₂CF₃-Gruppe oder einer -PO(OR^{b})₂-Gruppe, wobei **R**^{**b**} gleich einem Wasserstoffatom, einem gegebenenfalls substituierten aromatischen Carbozyklus oder Heterozyklus oder einer C1- bis C6-Alkylgruppe ist;
Rq ist gleich einer substituierten Pyridylgruppe, einer substituierten Pyrimidylgruppe, einer Phenylgruppe der Formel (VI) oder einem heterozyklischen Rest der Formel (VII) 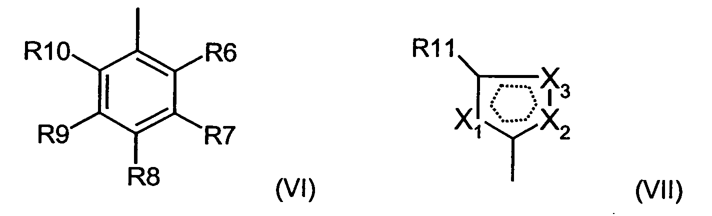 mit
**R6, R7, R8, R9** und **R10** sind unabhängig voneinander gleich einem Wasserstoffatom, einer geradkettigen oder verzweigten C1-C4-Alkylgruppe, einer C1-C4-Hydroxyalkylgruppe, einer Hydroxygruppe, einer Methoxygruppe, einer Benzylgruppe, einem Halogenatom, einer Nitrogruppe, einer Nitrosogruppe, einer Cyanogruppe, einer Trifluormethyl-gruppe, einer -CHO -Gruppe, einer -COR^{c}-Gruppe, einer -COOH -Gruppe, einer -CO₂R^{c}-Gruppe, einer -OCOR^{c}-Gruppe, einer -OCH₂Aryl-Gruppe, einer -SO₂NH₂-Gruppe, einer -NH₂-Gruppe, einer -(NH₃)⁺-Gruppe, einer -NHR^{c}-Gruppe, einer -(NH₂R^{c})⁺-Gruppe, einer -N(R^{c})₂-Gruppe, einer -(N(R^{c})₃)⁺-Gruppe, einer -NHCOR^{c}-Gruppe, einer -NHCOOR^{c}-Gruppe, einer -CH₂NH₂ -Gruppe, einer -CH₂NHR^{c}-Gruppe, einer -CH₂N(R^{c})₂-Gruppe, einer -CO₂CF₃-Gruppe, einer -PO(OR^{c})₂ -Gruppe, einer -SO₂CHF₂-Gruppe, einer -SO₂CF₃-Gruppe, einer -SO₂R^{c} - Gruppe oder einer -SR^{c}-Gruppe,
wobei **R**^{**c**} gleich einem Wasserstoffatom, einem gegebenenfalls substituierten aromatischen Carbozyklus oder Heterozyklus oder einer C1- C6-Alkylgruppe ist, mit der Massgabe, das zwei nebeneinanderliegende Reste R6 bis R10 auch gemeinsam mit den Kohlenstoffatomen des aromatischen Kerns einen 5- oder 6gliedrigen alizyklischen oder aromatischen Ring bilden können, der gegebenenfalls ein oder mehrere Schwefelatome, Stickstoffatome und/oder Sauerstoffatome enthalten kann; und
**X1** ist gleich Schwefel, Stickstoff, Sauerstoff, einer C-R13-Gruppe oder einer N-R12-Gruppe;
**X2** ist gleich Schwefel, Stickstoff, Sauerstoff, einer C-R14-Gruppe oder einer N-R12-Gruppe;
**X3** ist gleich Schwefel, Stickstoff, Sauerstoff, einer C-R15-Gruppe oder einer N-R12-Gruppe,
unter der Bedingung, dass mindestens einer und maximal zwei der Reste **X1** bis **X3** gleich Schwefel, Sauerstoff oder einer N-R12-Gruppe ist.
**R11, R13, R14** und **R15** sind unabhängig voneinander gleich Wasserstoff, einem Halogenatom (F, Cl, Br, J) einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄-Hydroxyalkylaminogruppe, einer Trifluormethangruppe, einer -C(O)CH₃-Gruppe, einer -C(O)CF₃-Gruppe, einer -Si(CH₃)₃-Gruppe, einer C₁-C₄-Hydroxyalkylgruppe oder einer C₃-C₄-Dihydroxyalkylgruppe; und
**R12** ist gleich Wasserstoff, einer C₁-C₆-Alkylgruppe, einer C₂-C₄-Hydroxyalkylgruppe, einer Phenylgruppe oder einer Acetylgruppe;
n ist gleich 0, 1 oder 2;
**Rx** ist gleich einem Wasserstoffatom, einer gegebenfalls substituierten Phenylgruppe, einer gegebenfalls substituierten Naphtylgruppe, einer Benzylgruppe, einem gegebenenfalls substituierten aromatischen Carbozyklus oder Heterozyklus, einer C1- bis C8-Polyhydroxyalkylgruppe, einer C1- bis C8-Alkoxy-(C1 bis C8)alkylgruppe, einer geradkettigen oder verzweigten C1-C6-Alkylgruppe, einer OR^{a}-Gruppe oder einer SR^{a}-Gruppe,
wobei **R**^{**a**} gleich einem Wasserstoffatom, einem gegebenenfalls substituierten aromatischen Carbozyklus oder Heterozyklus oder einer C1-C6-Alkylgruppe ist;
Rz ist gleich einem Wasserstoffatom, einer gegebenfalls substituierten Phenylgruppe, einer gegebenfalls substituierten Naphtylgruppe, einem gegebenenfalls substituierten aromatischen Carbozyklus oder Heterozyklus, einer C1- bis C8-Alkylgruppe, einer C1- bis C8-Monohydroxyalkylgruppe, einer C1- bis C8-Polyhydroxyalkylgruppe, einer C1- bis C8-Alkoxy-(C1 bis C8)alkylgruppe, einem Halogenatom, einer -OCOR^{a} -Gruppe, einer Nitrogruppe, einer Cyanogruppe, einer -CO-R^{a}-Gruppe, einer -CO-OR^{a}-Gruppe, einer -CO-OCF₃-Gruppe, einer -CO-NHR^{a}-Gruppe, einer -CO-N(R^{a})₂-Gruppe, einer -SO₂-NH₂-Gruppe, einer -SO₂-NHR^{a}-Gruppe, einer -SO₂-N(R^{a})₂, einer -SO₂-OR^{a}-Gruppe oder einer -SO₂-R^{a}-Gruppe, wobei **R**^{**a**} gleich einem Wasserstoffatom, einem gegebenenfalls substituierten aromatischen Carbozyklus oder Heterozyklus oder einer C1-C6-Alkylgruppe ist; und
**A**^{**-**} ist gleich dem Anion einer organischen oder anorganischen Säure; unter der Bedingung, dass **R**_{**q**} nicht gleich einer unsubstituierten Phenylgruppe oder unsubstituierten Thienylgruppe ist, wenn gilt **n**=0, **R1=R2=R3=R4=R5=Rz=H** und **Rx** gleich einer unsubstituierten Phenylgruppe.

2. Kationischer Farbstoff nach Anspruch 1, **dadurch gekennzeichnet, dass** er ausgewählt ist aus 4-[4-(4-N,N-dimethylamino)phenyl)-ethenyl]-7-hydroxy-2-phenyl-1-benzopyrylium-chlorid, 4-[4-(4-(N,N-dimethylamino)-phenyl)ethenyl]-7-methoxy-2-(3,4,5-trimethoxyphenyl)-1-benzopyryliumchorid und 4-[4-(4-(N,N-dimethyl-amino)phenyl)-butadienyl]-7-methoxy-2-(3,4,5-trimethoxy phenyl)-1-benzopyrylium-chlorid.

3. Verfahren zur Herstellung der kationischen Farbstoffe der Formel (V) gemäß Anspruch 1 oder 2, bei dem ein 4-Alkylen-2-aryl-4H-1-benzopyran-Derivat der Formel (la) oder dessen Salz der Formel (I) 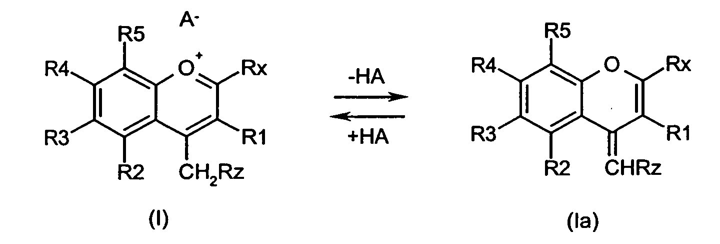 mit einem Aldehyd-Derivat der Formel (IV) bei 0 bis 180 °C in einem polar aprotischen, apolar aprotischen, polar protischen oder apolar protischen Lösungsmittel, umgesetzt wird; 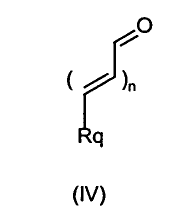 wobei gilt:
**R1, R2, R3, R4,** und **R5** sind unabhängig voneinander gleich einem Wasserstoffatom, einer geradkettigen oder verzweigten C1- bis C4-Alkylgruppe, einer C1- bis C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethylgruppe, einer -CHO -Gruppe, einer -COR^{b}-Gruppe, einer -COOH-Gruppe, einer -CO₂R^{b}-Gruppe, einer -OCOR^{b}-Gruppe, einer -OCH₂Aryl-Gruppe, einer -SO₂NH₂-Gruppe, einer -SO₂CHF₂-Gruppe, einer -SO₂CF₃-Gruppe, einer -SO₂NH₂-Gruppe, einer -SO₂NHR^{b}-Gruppe, einer -SO₂N(R^{b})₂-Gruppe, einer -SO₂R^{b}-Gruppe, einer -NH₂-Gruppe, einer -(NH₃)⁺-Gruppe, einer -NHR^{b}-Gruppe, einer -(NH₂R^{b})⁺-Gruppe, einer -N(R^{b})₂-Gruppe, einer -(N(R^{b})₃)⁺-Gruppe, einer -NHCOR^{b}-Gruppe, einer -NHCOOR^{b} -Gruppe, einer -CH₂NH₂-Gruppe, einer -CH₂NHR^{b}-Gruppe, einer -CH₂N(R^{b})₂-Gruppe, einer -CO₂CF₃-Gruppe oder einer -PO(OR^{b})₂ -Gruppe, wobei **R**^{**b**} gleich einem Wasserstoffatom, einem gegebenenfalls substituierten aromatischen Carbozyklus oder Heterozyklus oder einer C1- bis C6-Alkylgruppe ist;
**R**_{**q**} ist gleich einer substituierten Pyridylgruppe, einer substituierten Pyrimidylgruppe, einer Phenylgruppe der Formel (VI) oder einem heterozyklischen Rest der Formel (VII) 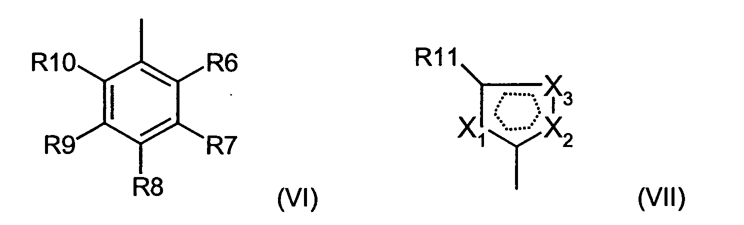 mit
R6, R7, R8, R9 und R10 sind unabhängig voneinander gleich einem Wasserstoffatom, einer geradkettigen oder verzweigten C1-C4-Alkylgruppe, einer C1-C4-Hydroxyalkylgruppe, einer Hydroxygruppe, einer Methoxygruppe, einer Benzylgruppe, einem Halogenatom (F, Cl, Br, J), einer Nitrogruppe, einer Nitrosogruppe, einer Cyanogruppe, einer Trifluormethyl-gruppe, einer -CHO -Gruppe, einer -COR^{c}-Gruppe, einer -COOH -Gruppe, einer -CO₂R^{c}-Gruppe, einer -OCOR^{c}-Gruppe, einer -OCH₂Aryl-Gruppe, einer -SO₂NH₂-Gruppe, einer -NH₂-Gruppe, einer -(NH₃)⁺-Gruppe, einer -NHR^{c}-Gruppe, einer -(NH₂R^{c})⁺-Gruppe, einer -N(R^{c})₂-Gruppe, einer -(N(R^{c})₃)⁺-Gruppe, einer -NHCOR^{c}-Gruppe, einer -NHCOOR^{c}-Gruppe, einer -CH₂NH₂-Gruppe, einer -CH₂NHR^{c}-Gruppe, einer -CH₂N(R^{c})₂-Gruppe, einer -CO₂CF₃ -Gruppe, einer -PO(OR^{c})₂-Gruppe, einer -SO₂CHF₂-Gruppe, einer -SO₂CF₃-Gruppe, einer -SO₂R^{c}- Gruppe oder einer -SR^{c}-Gruppe,
wobei **R**^{**c**} gleich einem Wasserstoffatom, einem gegebenenfalls substituierten aromatischen Carbozyklus oder Heterozyklus oder einer C1- C6-Alkylgruppe ist, mit der Massgabe, das zwei nebeneinanderliegende Reste **R6** bis **R10** auch gemeinsam mit den Kohlenstoffatomen des aromatischen Kerns einen 5- oder 6gliedrigen alizyklischen oder aromatischen Ring bilden können, der gegebenenfalls ein oder mehrere Schwefelatome, Stickstoffatome und/oder Sauerstoffatome enthalten kann; und
**X1** ist gleich Schwefel, Stickstoff, Sauerstoff, einer C-R13-Gruppe oder einer N-R12-Gruppe;
**X2** ist gleich Schwefel, Stickstoff, Sauerstoff, einer C-R14-Gruppe oder einer N-R12-Gruppe;
**X3** ist gleich Schwefel, Stickstoff, Sauerstoff, einer C-R15-Gruppe oder einer N-R12-Gruppe,
unter der Bedingung, dass mindestens einer und maximal zwei der Reste X1 bis X3 gleich Schwefel, Sauerstoff oder einer N-R12-Gruppe ist.
**R11, R13, R14** und **R15** sind unabhängig voneinander gleich Wasserstoff, einem Halogenatom (F, Cl, Br, J) einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxyalkyl)aminogruppe, einer C₁-C₄-Hydroxyalkylaminogruppe, einer Trifluormethangruppe, einer -C(O)CH₃-Gruppe, einer -C(O)CF₃-Gruppe, einer -Si(CH₃)₃-Gruppe, einer C₁-C₄-Hydroxyalkylgruppe oder einer C₃-C₄-Dihydroxyalkylgruppe; und
**R12** ist gleich Wasserstoff, einer C₁-C₆-Alkylgruppe, einer C₂-C₄-Hydroxyalkylgruppe, einer Phenylgruppe oder einer Acetylgruppe; **n** ist gleich 0, 1 oder 2;
**Rx** ist gleich einem Wasserstoffatom, einer gegebenfalls substituierten Phenylgruppe, einer gegebenfalls substituierten Naphtylgruppe, einer Benzylgruppe, einem gegebenenfalls substituierten aromatischen Carbozyklus oder Heterozyklus, einer C1- bis C8-Polyhydroxyalkylgruppe, einer C1- bis C8-Alkoxy-(C1 bis C8)alkylgruppe, einer geradkettigen oder verzweigten C1-C6-Alkylgruppe, einer OR^{a}-Gruppe oder einer SR^{a}-Gruppe,
wobei **R**^{**a**} gleich einem Wasserstoffatom, einem gegebenenfalls substituierten aromatischen Carbozyklus oder Heterozyklus oder einer C1-C6-Alkylgruppe ist;
Rz ist gleich einem Wasserstoffatom, einer gegebenfalls substituierten Phenylgruppe, einer gegebenfalls substituierten Naphtylgruppe, einem gegebenenfalls substituierten aromatischen Carbozyklus oder Heterozyklus, einer C1- bis C8-Alkylgruppe, einer C1- bis C8-Monohydroxyalkylgruppe, einer C1- bis C8-Polyhydroxyalkylgruppe, einer C1- bis C8-Alkoxy-(C1 bis C8)alkylgruppe, einem Halogenatom, einer -OCOR^{a}-Gruppe, einer Nitrogruppe, einer Cyanogruppe, einer -CO-R^{a}-Gruppe, einer -CO-OR^{a}-Gruppe, einer -CO-OCF₃-Gruppe, einer -CO-NHR^{a}-Gruppe, einer -CO-N(R^{a})₂-Gruppe, einer -SO₂-NH₂-Gruppe, einer -SO₂-NHR^{a}-Gruppe, einer -SO₂-N(R^{a})₂, einer -SO₂-OR^{a}-Gruppe oder einer -SO₂-R^{a}-Gruppe, wobei **R**^{**a**} gleich einem Wasserstoffatom, einem gegebenenfalls substituierten aromatischen Carbozyklus oder Heterozyklus oder einer C1-C6-Alkylgruppe ist; und
**A**^{**-**} ist gleich dem Anion einer organischen oder anorganischen Säure.

4. Verfahren nach Anspruch 3, **dadurch gekennzeichnet, dass** die Reaktionsdauer 1 bis 48 Stunden beträgt.

5. Verfahren nach Anspruch 3 oder 4, **dadurch gekennzeichnet, dass** das 4-Alkylen-2-aryl-4H-1-benzopyran-Derivat der Formel (Ia) oder dessen Salz der Formel (I) ausgewählt ist aus 7-Hydroxy-4-methylen-2-phenyl-4H-1-benzopyran, 7-Methoxy-4-methylen-2-(3,4,5-trimethoxyphenyl)-4H-1-benzopyran, 5,7-Dimethoxy-4-methylen-2-phenyl-4H-1-benzopyran, 4-Methylen-4H-1-benzopyran, 4-Methylen-2-phenyl-4H-1-benzopyran, 7-Amino-4-methylen-2-phenyl-4H-1-benzopyran, 7-Dimethylamino-4-methylen-2-phenyl-4H-1-benzopyran und 4-Methylen-7-nitro-2-phenyl-4H-1-benzopyran sowie deren Salze mit anorganischen oder organischen Säuren.

6. Verfahren nach einem der Ansprüche 3 bis 5, **dadurch gekennzeichnet, dass** das 4-Alkylen-2-aryl-4H-1-benzopyran-Derivate der Formel (la) oder dessen Salz der Formel (I) ausgewählt ist aus 7-Hydroxy-4-methyl-2-phenyl-1-benzopyrylium-chlorid, 7-Methoxy-4-methyl-2-(3,4,5-trimethoxyphenyl)-1-benzopyrylium-chlorid, 5,7-Dimethoxy-4-methyl-2-phenyl-1-benzopyrylium-chlorid, 4-Methyl-1-benzopyrylium-chlorid, 4-Methyl-2-phenyl-1-benzopyrylium-chlorid, 7-Amino-4-methyl-2-phenyl-1-benzopyrylium-chlorid, 7-Dimethylamino-4-methyl-2-phenyl-1-benzopyrylium-chlorid und 4-Methyl-7-nitro-2-phenyl-1-benzopyryliumchlorid.

7. Verfahren nach einem der Ansprüche 3 bis 6, **dadurch gekennzeichnet, dass** das Aldehyd-Derivat der Formel (IV) ausgewählt ist aus 4-N,N-Dimethylaminobenzaldehyd und 4-N,N-Dimethylaminocinnamaldehyd.

8. Mittel zur Färbung von Keratinfasern, **dadurch gekennzeichnet, dass** es in einer geeigneten Kosmetikgrundlage mindestens einen kationischen Farbstoff der Formel (V) gemäß Anspruch 1 oder 2 enthält.

9. Mittel nach Anspruch 8, **dadurch gekennzeichnet**, das der kationische Farbstoff der Formel (V) in einer Menge von 0,01 bis 5 Gewichtsprozent enthalten ist.

10. Mittel nach Anspruch 8 oder 9, **dadurch gekennzeichnet, dass** es ein Haarfärbemittel ist.
